# EUROPEAN PATENT APPLICATION

(11) **EP 1 005 878 A2**
(43) Date of publication of application: **07.06.2000**
(21) Application number: 99119035.6
(22) Date of filing: 29.09.1999
(51) Int. Cl.: A61M 16/16, A61M 16/08

(54) **System and method for determining changes in body resources during aided breathing**

(30) Priority: 01.12.1998 SE 9804148
(71) Applicant: Siemens-Elema AB, 171 95 Solna 1 (SE)
(72) Inventor: Krahbichler, Erik, 94315 Straubing (DE)

(57) **Abstract**

A system (11) for monitoring changes in a patient's body resources during breathing aided by a breathing apparatus (10) comprises means (8) adapted to determine the difference between corresponding values of either one or both of moisture content and temperature of each of an inspiration gas and an expiration gas within the breathing apparatus (10) as determined by moisture (5) and temperature (6) sensor elements within a common inspiration/expiration gas flow path (17); and processing means (7) adapted to process the determined difference to provide an output (9) related to the change in the associated body resource useable in the control of, for example, an inspiration gas pre-conditioning unit (16) to minimise the determined difference.

## Description

The present invention relates to breathing aid devices such as ventilators and respirators and in particular to a system and a method for determining changes in the body resources of a patient during aided breathing.

As used herein the term "breathing apparatus" shall be taken to mean a breathing aid device, such as a ventilator or a respirator, together with any associated control equipment and gas lines for delivering inspiration gas to and removing expiration gas from a patient.

During normal breathing conditions humans take in inspiration gas through their noses wherein the gas is warmed and moistened before being passed into the lungs. However, during aided breathing the respiratory gas is usually delivered into the lungs of a patient without passing through the nose. Energy is then expended by the patient in warming up the inspiratory gas and moisture is taken away from the patient in the expiratory gas. Such depletion of body resources (i.e. heat and moisture) may have undesirable consequences for the patient, especially for one who is already seriously ill or who's breathing is to be aided for extended periods.

In order to overcome this problem humidifiers and heaters may be included in the breathing apparatus in order to precondition inspiration gas by heating and moistening it. One such pre-conditioning apparatus is disclosed in US 5,383,447 and comprises a metal housing containing a passive heat and moisture exchanger; an active heat and moisture exchanger and a heating element. These components are designed to cooperate to provide an output of respiratory gas typically at a temperature of 36-37°C and 100% relative humidity (rh).

However, there remains no indication to an attending physician that the expenditure of the patient's body resources is such that humidification and heating of the inspiration gas is required or whether the temperature and rh of the delivered inspiration gas is correctly set within the aforementioned pre-conditioning apparatus so as not to place too great a strain on the respiratory system of an already sick patient.

It is an aim of the present invention to provide a system for determining changes in a patient's body resources (i.e. heat and moisture) and optionally the work required to be performed by the patient during assisted breathing which may be used to alleviate one or more of the problems associated with the prior art.

This is achieved by the system according to and characterised by claim 1. Thus by measuring one or both of the temperature and the moisture content of gases inspired and expired by a patient and determining their respective differences a signal related to the changes in a patient's associated body resource may be output by the system. This output may for example be used to drive a display unit to provide a visual display illustrative of the so determined differences.

Usefully, the output may be used as an a control signal for controlling components of a breathing apparatus such as a pre-conditioning apparatus to vary the moisture content and/or the temperature of the inspiration gas to be supplied to the patient in order to reduce the depletion of an associated body resource.

Usefully, the system may comprise a housing adapted to provide at least a section of a through-flow gas path that is common to both the inspiration and the expiration gases. A sensor having elements for measuring one or both temperature and moisture may then be housed in this common flow path.

This allows a single sensor to be used to make measurements in both the inspiration gas and the expiration gas.

Simply, a sensor may be used which is adapted to provide an output representative of the magnitude of the measured difference. Alternatively, the sensor may provide an output representative of the magnitude of the measured temperature and/or moisture and a separate calculations unit, for example within the processing means, may be provided. The calculations unit receives and holds the output from the sensor for each of the inspiration gas and the expiration gas and then determines their difference.

The invention will now be further described with reference to exemplifying embodiments illustrated in the drawings of the accompanying figures of which:
Figure 1 shows a schematic representation of a system according to the present invention.
Figure 2 shows a schematic representation of a breathing system including a system according to the present invention.

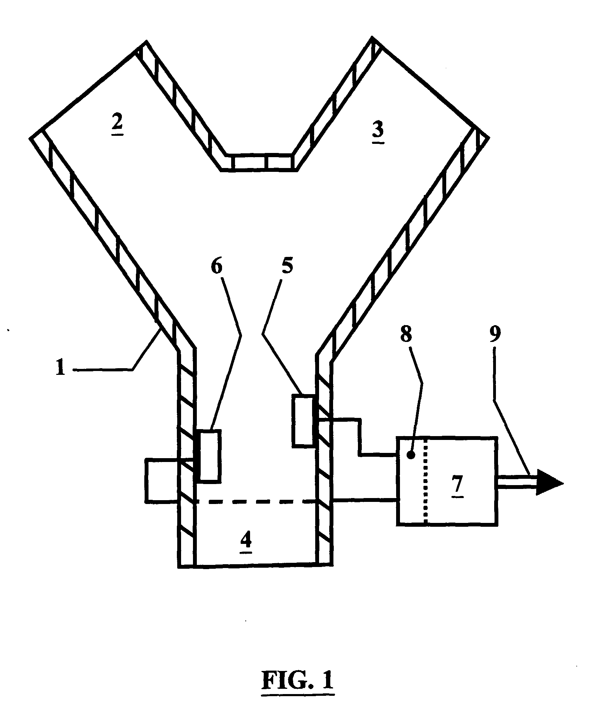

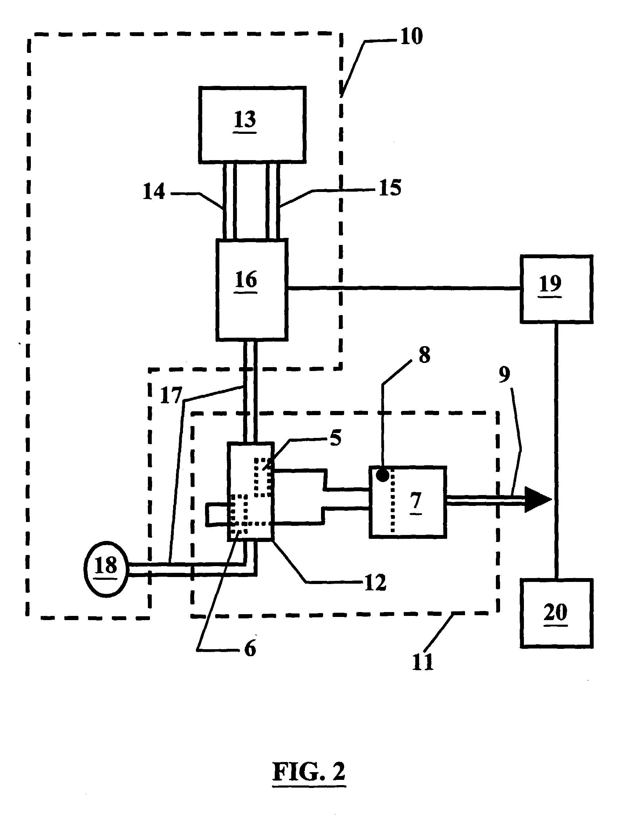

Considering Figure 1, a system according to the present invention for monitoring changes in a patient's temperature and moisture is shown. A Y-piece housing 1 provides a first branch 2 connectable to one or other of an inspiration line or an expiration line of a conventional breathing apparatus (shown as 10 in Figure 2) and a second branch 3 connectable to the other of the inspiration line or the expiration line. These branches 2,3 come together into a trunk 4 which forms a common flow path for inspiration and expiration gases.

A moisture sensor element 5 and a temperature sensor element 6 are located within the common gas flow path of the trunk 4 and have outputs operably connected to a computer 7. The sensor elements 5,6 are shown in Figure 1 as separate elements but may also be integrally formed on, for example a silicon substrate in a manner known in the art.

In use, the sensor elements 5,6 respectively measure moisture content and temperature of inspiration gas passing through the housing 1 from one of the branches 2 or 3. A signal representative of the magnitude of the measured parameter is output to and stored in a calculations unit 8 which is here formed as part of the computer 7 using conventional programming techniques known to those skilled in the art. Next, the sensor elements 5,6 respectively measure moisture content and temperature of expiration gas passing through the housing 1 from the trunk 4. Again a signal representative of the magnitude of the measured parameter is output to a calculations unit 8. The calculation unit 8 is suitably programmed to then determine the differences between the moisture content and temperature of the inspiration and the expiration gases. These differences and optionally the magnitudes of the measured parameters are then further processed by the computer 7 to provide an output signal 9 related to, for example indicative of, the change in an associated body resource of a patient to whom the breathing assistance is being provided. The computer 7 may additionally or alternatively be readily adapted to calculate the energy expended by the patient in warming the inspiration gas using information from the temperature sensor element 6.

The form of the output signal 9 depends on the intended use of that signal 9 and may for example be used to drive a conventional display unit to providing a visual representation indicative of the change in the patient's body resources during breathing or may be a control signal for use in controlling the operation of the breathing apparatus 10 such as that shown in Figure 2.

It is desirable to position the system of Figure 1 as close to the patient as possible without interfering with the normal operation of the breathing apparatus to which, in use it is connected. Therefore the trunk 4 may be connected to a face mask of a conventional breathing apparatus (for example the apparatus 10 of Figure 2), where it may substitute for the often used conventional Y-piece connector.

Figure 2 shows a representation of a breathing system which includes a conventional breathing apparatus 10 and a monitoring system 11 according to the present invention. In Figure 2 the housing 12 may be the Y-piece housing 1 of Figure 1 and components of the monitoring system 11 which are as shown in Figure 1 have the same reference numerals.

The breathing apparatus 10 comprises a ventilator 13 having an inspiration gas line 14 and an expiration gas line 15; a pre-conditioning apparatus 16, such as for example is disclosed in the above mentioned prior art document US 5,383,447, connected in-line to the gas lines 14,15 and to a common gas flow line 17. A gas mask 18, through which inspiration gas is delivered to a patient and expiration gas collected from a patient, is also included as a component of the breathing apparatus 10. The breathing apparatus 10 thus comprises components 13-18 all of which generally operate in a conventional manner and so will not be described in great detail herein.

The in-line housing 12, which may simply be a tubular connector or may be the Y-Piece connector 1 of Figure 1, forms a common flow path in which are located the moisture sensor element 5 and the temperature sensor element 6 of the monitoring system 11 of the present invention. The housing 12 is connected to common gas flow line 17 of the breathing apparatus 10 between the conditioning unit 16 and the face mask 18 and preferably as close to the face mask 18 as is practical. The housing 12 may be integral with the common line 17 or formed as a separate, removable, in-line unit.

In use the monitoring system 11 operates as described above with reference to Figure 1 and the output signal 9 from the computer 7 is used to provide control signals to a control unit 19 and to drive a display unit 20 which in this embodiment displays at least a graphical representation of the measured body resources expended by the patient.

The control unit 19 is operably connected to the preconditioning apparatus 16 to control the operation of active humidification and heating elements of the apparatus (16) to vary the moisture content and temperature of the inspiration gas to be supplied at the face mask 18 dependent on the determined differences obtained by the monitoring system 11 of the present invention so as to minimise the temperature difference between the inspired and the expired gas as well as the moisture content of the expired gas in order to lessen the expenditure of a patient's body resources.

The above embodiments of the present invention are described having sensors 5,6 that are arranged to measure the moisture and the temperature of both inspiration and expiration gases. It may be sufficient in some cases to arrange for the one or both sensors that are present to measure the relevant parameter only in the expiration gas. The calculations unit 8 may then be adapted to use otherwise presented values, for example from the control unit 19 or those manually input, for the moisture content and or temperature of the inspiration gas to provide alternative embodiments of the present invention whilst remaining within the scope of the invention as claimed.

## Claims

1. A system (11) for monitoring changes in a patient's body resources during breathing aided by a breathing apparatus (10) **characterised in that** the system comprises means (8) adapted to determine the difference between corresponding values of either one or both of moisture content and temperature of each of an inspiration gas and an expiration gas within the breathing apparatus (10); and processing means (7) adapted to process the determined difference to provide an output (9) related to the change in the associated body resource.

2. A system as claimed in claim 1 **characterised in that** the means (8) adapted to determine the difference comprises a sensor (5,6) adapted to measure one or both values of moisture content (5) and temperature (6) of each of the inspiration gas and the expiration gas within the breathing apparatus (10) and to output a signal dependent on the measured values.

3. A system as claimed in claim 2 **characterised in that** there is further provided a sensor housing (1,12) having therein a through-flow gas conduit (4,17) connectable to the breathing apparatus (10) to provide a common through-flow path for the inspiration gas and the expiration gas; and in that the sensor (5,6) is arranged in gas communication with the common flow path (4,17).

4. A system as claimed in claim 3 **characterised in that** the sensor (5,6) comprises a moisture sensor element (5) and a temperature sensor element (6) disposed within the common through-flow path gas conduit (4,17).

5. A breathing system comprising a breathing apparatus (10) having a mechanical breathing aid (13) for providing a controlled supply of an inspiration gas to a patient and a pre-conditioning apparatus (16) for varying one or both of the moisture content and the temperature of the inspiration gas **characterised in that** the breathing system further comprises a system (11) for monitoring the changes in the patient's body resources as claimed in any preceding claim wherein the processing means (7) is adapted to provide the output (9) to control the operation of the preconditioning apparatus (16) to vary one or both of the moisture content and temperature to lessen the depletion of the related body resource.

6. A method of monitoring changes in a patient's body resources during breathing aided by a breathing apparatus **characterised by** the steps of:
a) obtaining values of either one or both of moisture content and temperature of an inspiration gas and an expiration gas within the breathing apparatus;
b) determining the difference between corresponding values obtained from the inspiration gas and from the expiration gas; and
c) processing the determined difference to provide an output dependent on the change in the associated body resource.

7. A method as claimed in claim 6 **characterised in that** the step a) comprises the step of:
measuring, using sensor means both the water content and the temperature of each of the inspiration gas and the expiration gas at a site proximal the patient.
